# EUROPEAN PATENT APPLICATION

(11) **EP 4 678 731 A1**
(43) Date of publication of application: **14.01.2026**
(21) Application number: 24188302.4
(22) Date of filing: 12.07.2024
(51) Int. Cl.: C12N 1/16, C12N 1/18, C12P 23/00, C12P 39/00, A23K 10/37, A23K 20/174, A23K 20/179, A61K 8/31, A61K 8/34, A61K 8/35, A61K 8/67

(54) **METHOD OF FERMENTATION WITH YEAST STRAINS**

(71) Applicant: AlterBioTech Sp. z o.o., 50-302 Wroclaw (PL)
(72) Inventor: Pietka-Ottlik, Magdalena, 50-540 Wroclaw (PL); Krasowska, Anna, 51-163 Wroclaw (PL); Wrzesinska-Kolak, Dominika, 02-384 Warsaw (PL)
(74) Representative: Kondrat, Mariusz

(57) **Abstract**

The object of the invention is a method of fermentation with the yeast strains *Saccharomyces cerevisiae* and/or *Rhodotorulla bentica,* characterised by the fact that the fermentation takes place on a solid medium and that bakery and/or confectionery waste and the digest and extract from the digest obtained by supercritical carbon dioxide extraction (CO₂) are used as raw materials.

## Description

The subject of the invention is a method of fermentation with the yeast strains *Saccharomyces cerevisiae* and/or *Rhodotorulla bentica*, of bakery and/or confectionery waste, as well as the digest and the digest extract obtained by supercritical extraction with carbon dioxide (CO₂).

The bakery and confectionery market is very huge, only in 2020 Poles spent more than PLN 13 billion on confectionery. Approximately one third of this amount was on cakes and confectionery products. However, not all manufactured confectionery products are sold. The majority of them are returned to the manufacturer due to their expiry date. In light of the regulations, the manufacturer is obliged to hand them over to the relevant authorities for disposal or storage. This generates both high additional costs and a danger to the environment. Approximately 170,000 tonnes of products classified as bakery waste are produced in Poland every year. Although this forces the search for alternative means of disposal, contrary to EU recommendations, a large proportion of the waste still ends up in landfills. While bakery waste can be used, for example, for feed purposes provided it shows no signs of microbial infection (this forces the waste to be dried quickly to preserve it against microorganisms), confectionery waste is much more difficult to process due to its variety. This waste is characterised by its high energy value (high sugar content) and low fibre content.

There are known methods to obtain carotenoids, including astaxanthin through the use of microbial cultures. To date, the yeast strain *Phaffia rhodozyma* has been used in cultures on mixtures of organic acids and mono-, di-, and polysaccharides. Fermentation of feed materials with the yeast strains *Phaffia rhodozyma* and *Sporobolomyces roseus* has been shown to enrich these materials in carotenoids. To date, bakery and confectionery waste has not been revealed as a fermentable object for the extraction of carotenoids, supplementary feed mixtures (MPUs) or carotenoid-enriched feed additives.

From document EP0367765, a method for obtaining astaxanthin by fermentation using a strain of *Phaffia rhodozyma* is known. The yeast cells are cultured by batch fermentation, which is carried out under appropriate aeration, the batch fermentation comprising a growth phase and a subsequent limited growth phase. After culture, astaxanthin can be extracted supercritically from the yeast cells. The carbon source presented in the fermentation carried out can be molasses or sucrose.

A method for obtaining astaxanthin using the bacteria strain *Phaffia rhodozyma* is known from document EP0454024. The carbon sources in this process can be succinate, malate, pyruvate, glucose, sucrose, fructose, maltose, corn syrup, hydrolysed starch and combinations of two or more of these.

The essence of the invention is a method of fermentation with yeast strains *Saccharomyces cerevisiae* and/or *Rhodotorulla bentica*, characterised in that the fermentation takes place on a solid medium and bakery and/or confectionery waste is used as raw materials. Preferably, solid-state fermentation takes place at a temperature of ~18 to 28 °C over a period of at least 16 hours with continuous aeration of the fermentation mass at a rate of 0.01 to 10 air volumes per bioreactor volume per 1 min.

Preferably, fermentation is carried out with the addition of LAB - *Lactococcus lactis* bacteria.

Preferably, in the first fermentation stage, the bakery or confectionery waste is pre-pasteurised or milled and then pasteurised.

Preferably, the antioxidant substances obtained by fermentation are vitamins, advantageously tocopherol; antioxidants, advantageously astaxanthin, zeaxanthin, their isomers, β-carotene; natural pigments or lactic acid.

Another subject is the digest obtained according to the method described above characterised by the fact that it is used as an additive in animal feed.

Preferably, the digest is fluid dried, mixed with meal as a feed base ingredient, advantageously soybean meal, at a maximum temperature of 60 °C with air passing through the dried material from bottom to top.

Preferably, the digest contains protein in an amount of up to 10 % by weight, fat in an amount of up to 30 % by weight, and polyphenols in an amount of at least 0.40 % by weight, vitamin E in an amount of at least 0.07 µg/g, astaxanthin in an amount of at least 0.003 µg/g, zeaxanthin in an amount of at least 0.006 µg/g and β -carotene in an amount of at least 0.060 µg/g.

The digest obtained according to the method described above is extracted with carbon dioxide (CO₂) in a supercritical state.

Preferably, the extraction of the digest is carried out at a temperature of 35 - 50 °C, at a pressure in the range of 10-40 MPa, preferably 25 MPa, preferably with a carbon dioxide flow rate (CO₂) of 2 L/min or with ethanol or isopropanol cosolvents at a pressure of 40 MPa.

Preferably, the digest extract obtained by the method described above contains vitamin E in an amount of min. 10.5 µg/g, and/or β -carotene in an amount of min. 1 µg/g, astaxanthin in an amount of min.0.5 µg/g and zeaxanthin in an amount of min. 1 µg/g, polyphenols in an amount of min. 0.473 mg/g.

The aim of the invention was to develop a waste-free method for the microbiological biotransformation of bakery and confectionery waste.

The following products were obtained:
- an extract with antioxidant activity due to its content of mainly carotenoids;
- a high-added-value digest obtained by enrichment with health-promoting substances through microbial fermentation, which can be a feed additive.

The method according to the invention uses solid state fermentation (ssf) technology using appropriately selected yeast-bacteria cocultures and yields a product containing antioxidants, including vitamin E and the carotenoids astaxanthin, zeaxanthin and their isomers. It should be emphasised that the method according to the invention is a waste-free process. The management of bakery and/or confectionery waste, is a major environmental problem. It is possible to obtain antioxidants, among them vitamin E or carotenoids: astaxanthin, zeaxanthin, etc.

By using appropriate cocultures of micro-organisms, it is possible to produce valuable compounds such as carotenoids, which include vitamins (α-tocopherol), antioxidants (astaxanthin, zeaxanthin, β-carotene), natural pigments or lactic acid. Carotenoids have health-promoting properties and are used as additives in food products or cosmetics. They are also used with enhancers (boosters) in the form of plant extracts, e.g. from clove (*Eugenia caryophyllus*) or bearberry (*Arctostaphylos uva-ursi*).

The advantage of the solution is the waste-free processing of the currently troublesome bakery and confectionery waste into products with useful values. For example, extracts with strong antioxidant and anti-ageing properties can be produced as a cosmetic additive, as well as digestates with health-promoting properties, resulting in faster fattening of animals (e.g. broilers), used as a feed additive.

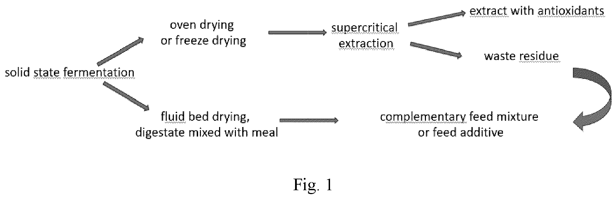

The invention is illustrated in the following worked examples and in the drawing, in which fig. 1 shows a scheme for the fermentation of bakery and confectionery waste on a solid substrate (ssf).

### Example 1

Strains used for ssf fermentation: yeasts *Saccharomyces cerevisiae* and *Rhodotorulla bentica*, LAB bacteria - *Lactococcus lactis.*

Fermentation parameters: a typical bioreactor was used for the process, e.g. 50 L bioreactor for ssf processes, manufacturer InventionBio S.A. known from patent Pat.238781, liquid medium for the culture of microorganisms (preparation of the culture for inoculation of bakery and/or confectionery waste) consisting of molasses (2%), yeast extract (1%) plus demineralised sterile water. The culture was conducted at room temperature (~18 - 28 °C), for 16 - 20 hours, using stirred yeast for aeration. Different types of food materials were used, including crusty material (MK), cream material (K) (stored at 4 °C before all processes), which were milled separately. The waste material in the form of MK and K was pasteurised for half an hour before inoculation with microorganisms and the start of ssf fermentation separately for each material. The ssf fermentation lasted at least 16 hours, at a temperature of ~ 18 to 28 °C with continuous aeration by stirring. No humidification or additional feed of raw material into the bioreactor was used. Depending on the moisture content, the resulting digest was dried in an oven, on trays, or by freeze-drying.

### Example 2

Use of the digestate obtained by the method according to the invention as a feed ingredient for animal nutrition.

Fermentation of the ssf was carried out as in Example 1 and a digestate was obtained, which was mixed with an element of future animal feed, e.g. soybean meal (depending on the moisture content of the digestate at a ratio of 1:1 or 2:1 (digestate:meal)) and fluidised bed drying was applied.

Table 1 shows the carotene content:

**Table 1.**

| **Carotenes + vit E** | **Concentration range [µg/g of digest].** |
|---|---|
| Vit E | 0.07 - 0.22 |
| Astaxanthin | 0.003 - 0.02 |
| Zeaxanthin | 0.0066 - 0.08 |
| b-carotene | 0.0066 - 0.022 |

Table 2 shows the contents of the various components of the digestates:

**Table 2**

| **Sample** | **Calcula ted protein %** | **Crude fat %** | **Kiejldahl nitrogen %** | **Total ash %** |
|---|---|---|---|---|
| MK | 7.92 | 11.34 | 1.267 | 2.18 |
| K | 6.69 | 29.90 | 1.07 | 2.38 |
| fermented MK | 8.15 | 11.87 | 1.305 | 2.33 |
| fermented K | 6.35 | 26.87 | 1.015 | 2.85 |

### Example 3

Fermentation of the ssf was carried out as in Example 1 and the digestate was obtained, which was dried by freeze-drying and then subjected to supercritical extraction with CO₂ at 40 °C, under 25 MPa pressure, CO₂ flow 2 L/min.

The extract obtained is used to make cosmetics. The extract is enriched with antioxidant substances.

The extract was tested for carotenoids, vitamin E and polyphenols. The extracts have a high antioxidant activity, measured by a method using DPPH (2,2-diphenyl-1-picrylhydrazyl), at the level of the synthetic antioxidant Trolox, added to some cosmetics. The extracts obtained by the method according to the invention are of entirely natural origin.

Of the carotenoids, astaxanthin and its isomers, zeaxanthin and its isomers, cryptoxanthin, antheraxanthin and b-carotene were detected. Table 3 shows the carotene and vitamin E contents of the extracts. The MK fermentation extract contained 0.473 mg/g polyphenols and the K fermentation extract 0.431 mg/g.

In addition, a booster - bearberry macerate in a ratio of 1:8 (1 part bearberry to 8 parts extract obtained from MK or K waste) was added to the extracts.

**Table 3.**

| **Carotenes + vit E** | **Concentration range [µg/g of extracts]** |
|---|---|
| Vit E | 10.5 - 33 |
| Astaxanthin | 0.5-3 |
| Zeaxanthin | 1 - 12.5 |
| b-carotene | 1-3.3 |

### Example 4

Study of feeding broiler chickens with digestates obtained by biotransformation of MK and K waste according to the invention.

Growth studies were carried out on 200 slaughter chicks of the Ross 308 line. The day-old chicks were inserted into 5 pre-prepared enclosures with 4 boxes each provided with a drinker and a feeder. The chicks were divided into 5 experimental groups, while each group consisted of 4 replicates. The chicks were maintained under controlled temperature and humidity conditions, under heat lamps, according to the genetic line manufacturer's guidelines (Aviagen, Manual 2022). For the first 10 days, the birds received a starter feed mixture without test formulations. The experimental diets admixed with the digest obtained in example 2 were introduced from the grower feed mixture (day 14 of life) and were used until the end of the experiment (day 35 of the birds' life).

After 35 days of the experiment, the chickens had a significantly higher body weight compared to the chickens in the control groups, with the confectionery waste itself in its unprocessed form being unsuitable for feeding to poultry as a component of compound feeds.

### Example 5

The extract obtained as in example 3 was used as an ingredient in a cosmetic composition with the qualitative composition described below:
water, grape seed oil, glycerin, Butyrospermum Parkii butter, cetearyl olivate, EXTRACT K or MK, sorbitan olivate, cetearyl alcohol, squalane, hydrogenated ethylhexyl olivate, olive oil, corn starch, glycine soybean oil, sodium phytate, hydrogenated olive oil, unsaponifiable, tocopherol, beta-sitosterol, citric acid, squalene, phenoxyethanol, ethylhexylglycerin, fragrance.

### Example 6

Cosmetic masses containing extracts (obtained from MK and K separately) were created and such pilot creams were tested for their moisturising properties, effect on skin pH, skin smoothing, reduction of wrinkle length and depth, reduction of skin sensitivity, and increase of skin density.

Tables 4, 5, 6 show the results of the apparatus tests of the cream with K extract, MK extract and without extract against the parameters analysed respectively, such as:
1. Skin hydration - the test was carried out on a Corneometer^{®} CM 825 - the aim of the test was to evaluate the direct effect of the product on the level of skin hydration, using the capacitive method and single mode. The depth of penetration ranged from 10 µm to 20 µm in the stratum corneum. The higher the value, the more moisturised the skin.
2. Skin pH - the test was carried out on a Skin-pH-meter^{®} PH905 instrument - the aim of the test was to assess the direct effect of the product on the skin's pH level.
3. Skin smoothing - the test was performed on a Visioscan^{®} VC 20plus instrument - the aim of the study was to assess the direct effect of the product on skin topography. The method used was *SELS (Surface Evaluation of the Living Skin*), the *SEsm* parameter being qualitatively and quantitatively responsible for the physiological state of skin smoothness. The lower the value of the SEsm parameter, the greater the skin smoothness.
4. Skin elasticity - the test was performed on a Cutometer^{®} dual MPA 580 - the purpose of the test was to measure the viscoelastic properties of the skin, under negative pressure causing mechanical deformation of the skin by suction into the probe gap. The test was conducted in mode 1, at a pressure of 450 mbar and a probe diameter of 2 mm. An interpretation of the parameter R7 was used to measure skin elasticity, the higher the value, the higher the skin elasticity.
5. Skin firmness - the test was carried out on a Cutometer^{®} dual MPA 580 apparatus - the purpose of the test was to measure the viscoelastic properties of the skin, under negative pressure causing mechanical deformation of the skin by sucking it into the probe slot. The test was conducted in mode 1, at a pressure of 450 mbar and a probe diameter of 2 mm. An interpretation of the parameter R0 was used to measure skin firmness, the lower the value, the higher the skin firmness.
6. Skin density - the test was performed on an Ultrascan UC 22 - the aim of the test was to measure skin density using 22MHz ultrasound waves. Skin penetration depth - 6-8mm.
7. Skin sensitivity - the test was performed on an ASW 300F 30x camera - the aim of the test was to determine the effect of the product on skin sensitivity. The test was performed using light microscopy at 30x magnification of the blood vessels. A scale of 1-100 was used, with 1 being the least sensitive and 100 the most sensitive.
8. Length and depth of wrinkles - the study was performed on a Visioline^{®} VL650 Quantirides^{®} apparatus - the aim of the study was to evaluate the anti-ageing effect of the product. Wrinkles were measured using silicone replicas and interpretation of the parameters length (mm) and depth (µm). The smaller the numerical value, the shallower and shorter the wrinkles.

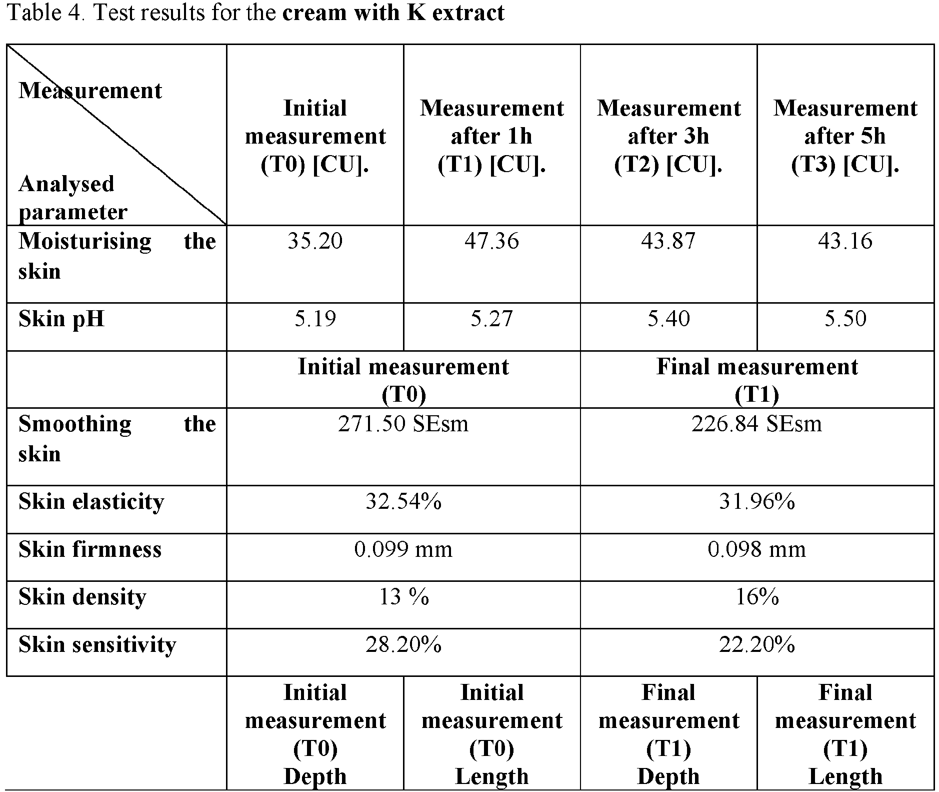

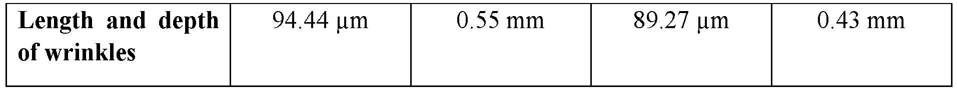

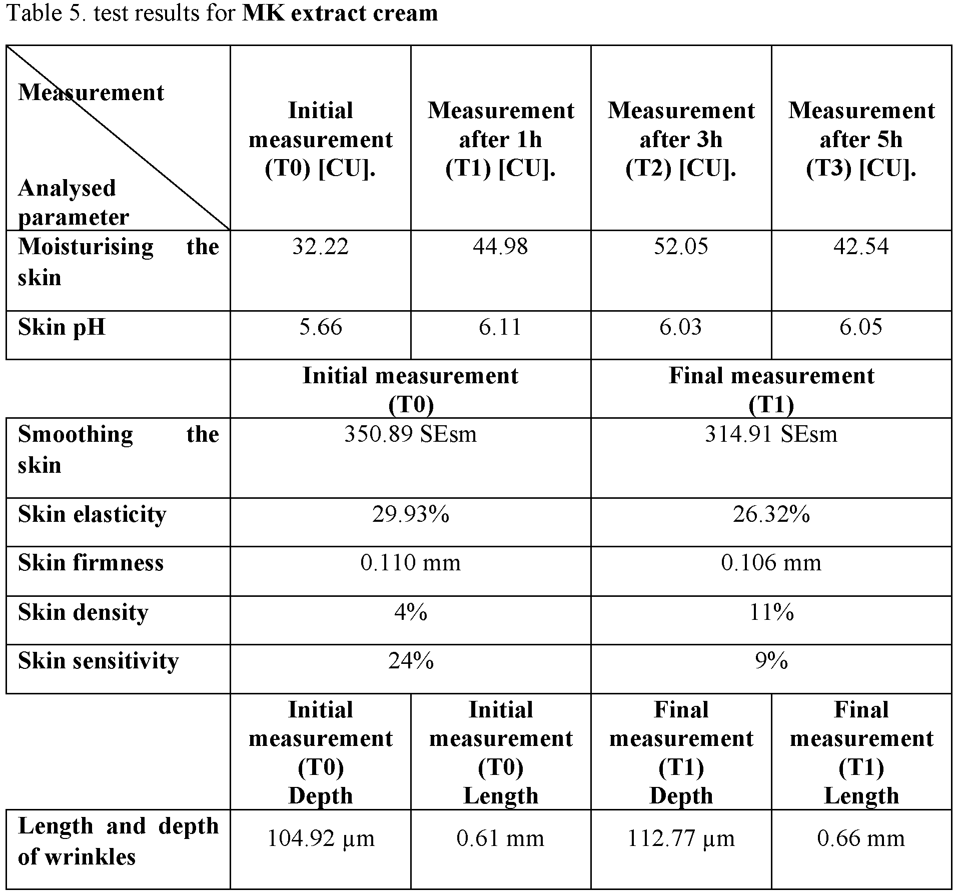

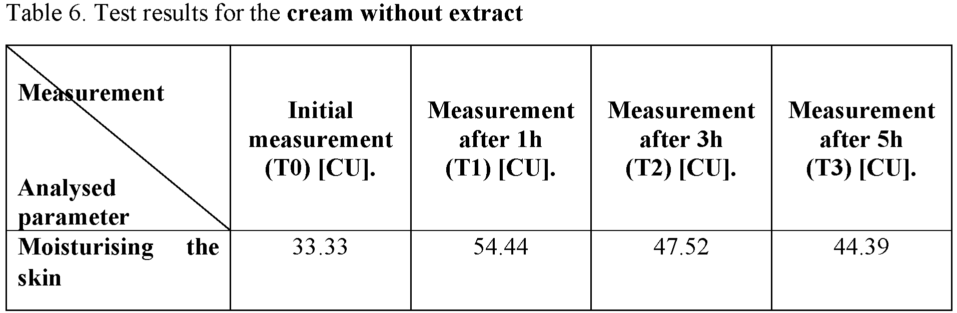

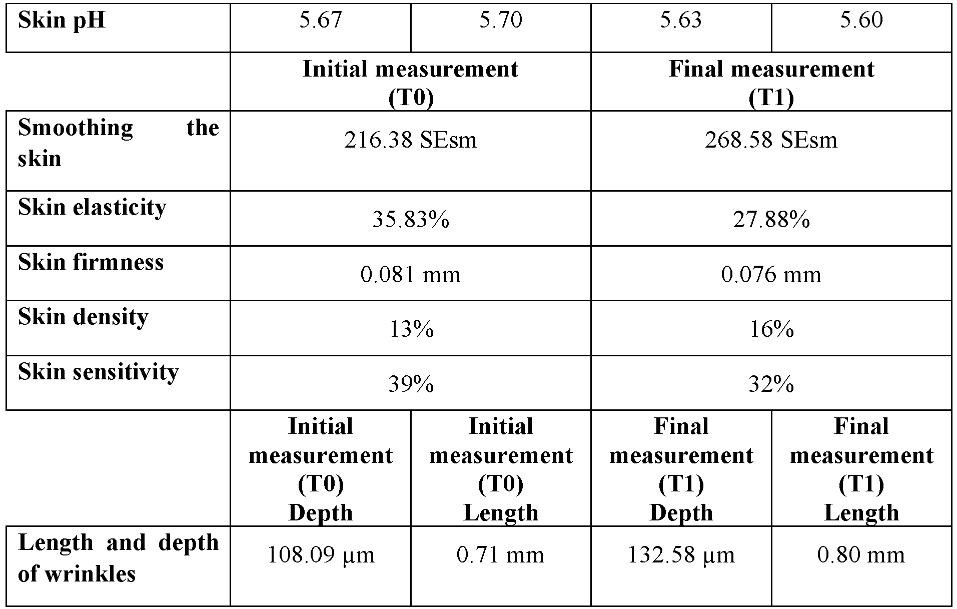

Research has shown that cosmetics exhibit different properties depending on the extract contained (MK or K). Cream with K extract: moisturises the skin, maintains a slightly acidic skin pH, smooths the skin, reduces the length and depth of wrinkles, reduces skin sensitivity, increases skin density. MK extract cream moisturises the skin, smoothens the skin, reduces skin sensitivity, increases skin density, increases skin firmness.

## Claims

1. Method of fermentation with yeast strains *Saccharomyces cerevisiae* and/or *Rhodotorulla bentica,* **characterised by the fact that** the fermentation takes place on a solid substrate and bakery and/or confectionery waste is used as raw materials.

2. The method according to claim 1, **characterised in that** solid-state fermentation occurs at a temperature of ~18 to 28 °C over a period of at least 16 hours with continuous aeration of the fermentation mass at a rate of 0.01 to 10 air volumes per bioreactor volume per 1 min.

3. The method according to claim 1 or 2, **characterised in that the** fermentation is carried out with the addition of the bacterium LAB - *Lactococcus lactis.*

4. The method according to claim 1, **characterised in that** in a first step the bakery or confectionery waste is pre-pasteurised or ground and then pasteurised.

5. The method according to claim 1, **characterised in that the** antioxidant substances obtained by fermentation are vitamins, preferably tocopherol; antioxidants, preferably astaxanthin, zeaxanthin, their isomers β-carotene; polyphenols; natural colouring agents or lactic acid.

6. The digestate obtained according to the method described in Claim. 1, **characterised in that** it is used as an additive to the animal feed mixture.

7. The digest according to claim. 6 **characterised by** the fact that it is fluidly dried, in a mixture with a meal as a feed base ingredient, preferably soybean meal, at a maximum temperature of 60° C by means of air passing through the dried material from bottom to top.

8. Ferment according to claim. 6, **characterised in that** it contains protein in an amount of up to 10 % by weight, fats in an amount of up to 30 % by weight, and polyphenols in an amount of at least 0.40 % by weight, vitamin E in an amount of at least 0.07 µg/g, astaxanthin in an amount of at least 0.003 µg/g, zeaxanthin in an amount of at least 0.006 µg/g and β-carotene in an amount of at least 0.006 µg/g.

9. The digestate obtained according to the method described in claims. 1 -5, **characterised in that** it is extracted with carbon dioxide (CO₂) in a supercritical state.

10. Ferment according to claim. 9 **characterised by** the fact that extraction is carried out at a temperature of 35 - 50 °C, at a pressure in the range of 10-40 MPa, preferably 25 MPa, preferably with a carbon dioxide flow rate (CO₂) of 2 L/min or with cosolvents of ethanol or isopropanol at a pressure of 40 MPa.

11. A digest extract obtained by the method described in claims. 9 or 10, **characterised in that** it contains vitamin E in an amount of min. 10.5 µg/g, and/or β-carotene min. 1 µg/g, astaxanthin min. 0.5 µg/g and zeaxanthin min. 1 µg/g, polyphenols min. 0.473 mg/g.
